(19) Europäisches Patentamt
European Patent Office
Office européen des brevets

(11) **EP 4 239 644 A1**

(12) **EUROPEAN PATENT APPLICATION**

(43) Date of publication:
**06.09.2023 Bulletin 2023/36**

(21) Application number: **22172557.5**

(22) Date of filing: **10.05.2022**

(51) International Patent Classification (IPC):
**G16H 30/20** (2018.01)  **G16H 30/40** (2018.01)

(52) Cooperative Patent Classification (CPC):
**G16H 30/20; G16H 30/40**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.03.2022 PCT/CN2022/079263**

(71) Applicant: **Koninklijke Philips N.V.
5656 AG Eindhoven (NL)**

(72) Inventors:
• **TAO, Liang
  Eindhoven (NL)**
• **LI, Zuofeng
  Eindhoven (NL)**

(74) Representative: **Philips Intellectual Property &
Standards
High Tech Campus 52
5656 AG Eindhoven (NL)**

(54) **CROSS-MODALITY DATA MATCHING**

(57)     Embodiments of the present disclosure relate to cross-modality data matching. Some embodiments of the present disclosure provide a method for medical data validation. The method comprises constructing a data matching model to be configured to determine whether an input data sample matches with a reference data sample included in either one of a first dataset of a first type and a second dataset of a second type; and training the data matching model with a first and a second training sample sets according to a training objective, the training objective configured to enable the trained data matching model to determine that the first positive data sample matches with and the first negative data sample mismatches with the first anchor data sample, and to determine that the second positive data sample matches with and the second negative data sample mismatches with the second anchor data sample.

Fig. 2

EP 4 239 644 A1

**Description**

FIELD

Embodiments of the present disclosure generally relate to the field of automated data analysis technologies and in particular, to a method, system, and computer storage medium for cross-modality data matching.

BACKGROUND

It is useful in many applications to use data in one modality to search for matching data in a different modality. An example of such cross-modality data matching is to input an image to search for a text document describing the image. In this case, the image and the text document are considered as data in two different modalities. Another example for the cross-modality data matching is to match an image of a certain style with another image of a different style.

The cross-modality data matching is useful in the medical field. There may be a huge amount of medical imaging data sources, but the associated text reports are not often ready to be available for the patients or physicians. Further, writing reports is a tedious and time-consuming work for experienced radiologists and physicians, whereas it is rather a challenge for young and junior radiologists and physicians to quickly write the high-quality reports for medical imaging data. In some cases, a radiologist may need to read hundreds of clinical images per workday and write reports about the clinical images. Considering such a case, it is desired to facilitate writing of reports about medical imaging. Even if the reports cannot be automatically produced and issued due to the complicated nature of medical imaging data, it would be helpful to reduce time and improve the quality by recommending some existing high-quality reports about medical images that are matched with an input medical image.

With the development of artificial intelligence (AI) technologies, it is possible to train a model to implement various tasks including data matching by means of deep learning. However, there is not found a robust, accurate, and fast solution to perform the cross-modality data matching task.

SUMMARY

In general, example embodiments of the present disclosure provide a solution for cross-modality data matching.

In a first aspect, there is provided a computer-implemented method. The method comprises constructing a data matching model to be configured to determine whether an input data sample matches with a reference data sample included in either one of a first dataset of a first type and a second dataset of a second type; obtaining a first training sample set comprising a first anchor data sample of a first type, and a first positive data sample and a first negative data sample of a second type; obtaining a second training sample set comprising a second anchor data sample of the second type, and a second positive data sample and a second negative data sample of the first type; and training the data matching model with the first and second training sample sets according to a training objective, the training objective configured to enable the trained data matching model to determine that the first positive data sample matches with and the first negative data sample mismatches with the first anchor data sample, and to determine that the second positive data sample matches with and the second negative data sample mismatches with the second anchor data sample.

In a second aspect, there is provided a computing system. The computing system computing system comprises at least one processor; and at least one memory comprising computer readable instructions which, when executed by the at least one processor of the electronic device, cause the electronic device to perform acts comprising: constructing a data matching model to be configured to determine whether an input data sample matches with a reference data sample included in either one of a first dataset of a first type and a second dataset of a second type; obtaining a first training sample set comprising a first anchor data sample of a first type, and a first positive data sample and a first negative data sample of a second type; obtaining a second training sample set comprising a second anchor data sample of the second type, and a second positive data sample and a second negative data sample of the first type; and training the data matching model with the first and second training sample sets according to a training objective, the training objective configured to enable the trained data matching model to determine that the first positive data sample matches with and the first negative data sample mismatches with the first anchor data sample, and to determine that the second positive data sample matches with and the second negative data sample mismatches with the second anchor data sample.

In a third aspect, there is provided a computer readable medium. The computer readable medium comprises instructions which, when executed by at least one processor, cause the at least one processor to perform the method of the first aspect.

It is to be understood that the summary section is not intended to identify key or essential features of embodiments of the present disclosure, nor is it intended to be used to limit the scope of the present disclosure. Other features of the present disclosure will become easily comprehensible through the following description.

BRIEF DESCRIPTION OF THE DRAWINGS

The following detailed description of the embodiments of the present disclosure can be best understood when read in conjunction with the following drawings, where:

Fig. 1 illustrates an example environment in which embodiments of the present disclosure may be implemented.
Fig. 2 illustrates a block diagram of architecture of a data matching model according to some embodiments of the present disclosure.
Fig. 3 illustrates an example of constructing training sample sets for training the data matching model according to some embodiments of the present disclosure.
Fig. 4 illustrates a block diagram of the model training system for training the data matching model according to some embodiments of the present disclosure.
Fig. 5 illustrates a block diagram of the model training system for training the data matching model according to some other embodiments of the present disclosure.
Fig. 6 illustrates a block diagram of the model applying system for performing data matching according to some embodiments of the present disclosure.
Fig. 7 illustrates a flowchart of an example method according to some embodiments of the present disclosure.
Fig. 8 illustrates a flowchart of an example method according to some other embodiments of the present disclosure; and
Fig. 9 illustrates a block diagram of an example computing system/device suitable for implementing example embodiments of the present disclosure.

Throughout the drawings, the same or similar reference numerals represent the same or similar element.

DETAILED DESCRIPTION

Principle of the present disclosure will now be described with reference to some embodiments. It is to be understood that these embodiments are described only for the purpose of illustration and help those skilled in the art to understand and implement the present disclosure, without suggesting any limitation as to the scope of the disclosure. The disclosure described herein can be implemented in various manners other than the ones described below.

In the following description and claims, unless defined otherwise, all technical and scientific terms used herein have the same meaning as commonly understood by one of ordinary skills in the art to which this disclosure belongs.

References in the present disclosure to "one embodiment," "an embodiment," "an example embodiment," and the like indicate that the embodiment described may include a particular feature, structure, or characteristic, but it is not necessary that every embodiment includes the particular feature, structure, or characteristic. Moreover, such phrases are not necessarily referring to the same embodiment. Further, when a particular feature, structure, or characteristic is described in connection with an example embodiment, it is submitted that it is within the knowledge of one skilled in the art to affect such feature, structure, or characteristic in connection with other embodiments whether or not explicitly described.

It shall be understood that although the terms "first" and "second" etc. may be used herein to describe various elements, these elements should not be limited by these terms. These terms are only used to distinguish one element from another. For example, a first element could be termed a second element, and similarly, a second element could be termed a first element, without departing from the scope of example embodiments. As used herein, the term "and/or" includes any and all combinations of one or more of the listed terms.

The terminology used herein is for the purpose of describing particular embodiments only and is not intended to be limiting of example embodiments. As used herein, the singular forms "a", "an" and "the" are intended to include the plural forms as well, unless the context clearly indicates otherwise.

It will be further understood that the terms "comprises", "comprising", "has", "having", "includes" and/or "including", when used herein, specify the presence of stated features, elements, and/or components etc., but do not preclude the presence or addition of one or more other features, elements, components and/ or combinations thereof.

As used herein, the term "model" is referred to as an association between an input and an output learned from training data, and thus a corresponding output may be generated for a given input after the training. The generation of the model may be based on a machine learning technique. Deep learning (DL) is one of the machine learning algorithms which processes an input and provide the corresponding output using processing units in multiple layers. As used herein, "model" may also be referred to as "machine learning model", "learning model", "machine learning network", or "learning network," which are used interchangeably herein.

Neural network (NN) model is an example deep learning model. A neural network can process an input and provides a corresponding output and it generally includes an input layer, an output layer and one or more hidden layers between the input and output layers. The neural network used in the deep learning applications generally includes a plurality of hidden layers to increase the depth of the network. Individual layers of the neural network model are connected in sequence, such that an output of a preceding layer is provided as an input for a following layer, where the input layer receives the input of the neural network while the output of the output layer acts as the final output of the neural network. Each layer of the neural network includes one or more nodes (also referred to as processing nodes or neurons), each of which processes the input from the preceding layer.

Generally, machine learning may include three stages, i.e., a training stage, a test stage, and an application stage (also referred to as an inference stage). In the training stage, a given machine learning network may be trained iteratively using a great amount of training data until the network can obtain, from the training data, consistent inference similar to those that human intelligence can make. Through the training, the machine learning network may be regarded as being capable of learning the association between the input and the output (also referred to an input-output mapping) from the training data. The set of parameter values of the trained network is determined. In the test stage, a test input is applied to the trained model to test whether the machine learning network can provide a correct output, so as to determine the performance of the network. In the application stage, the machine learning network may be used to process an actual network input based on the set of parameter values obtained in the training and to determine the corresponding network output.

*Working Principle and Example Environment*

According to example embodiments of the present disclosure, there is proposed an improved solution for cross-modality data matching. In this solution, a machine learning model, called a data matching model, is constructed to implement the cross-modality data matching. To improve accuracy and efficiency of the cross-modality data matching, the data matching model is trained with data samples of a first type and a second type to meet a certain training objective. Through the training process, an efficient and advanced data matching model can be offered for an input data sample of any of the two types. The data matching model can be used to determine matching data samples of any of the two types for the input data sample regardless the type of the input data sample. As such, it is feasible to obtain desired matching data results by providing input data samples of either of the two types.

In the following, example embodiments of the present disclosure are described with reference to the drawings.

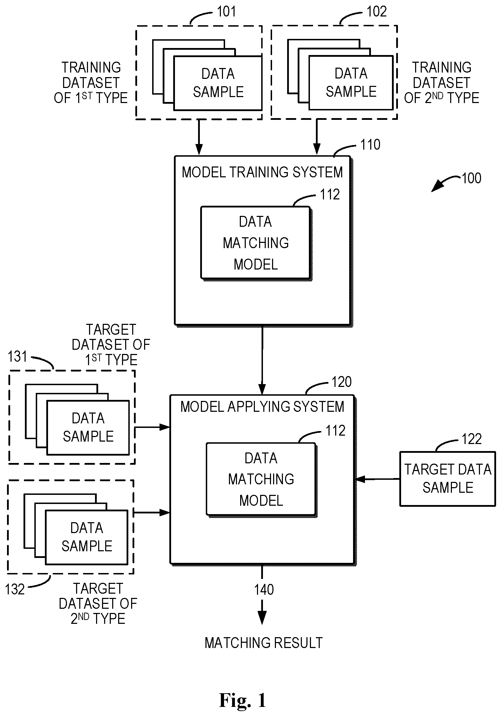

Fig. 1 illustrates an example environment 100 in which embodiments of the present disclosure may be implemented. The environment 100 comprises a model training system 110 and a model applying system 120. It would be appreciated that although being illustrated separately, the model training system 110 and the model applying system 120 can be implemented as a single physical device or system to perform their functionalities described herein. In some embodiments, the model training system 110 and/or the model applying system 120 may include any system or device having the computing capability, such as computing systems, mainframes, servers, edges devices, and/or the like. The scope of the present disclosure is not limited in this regard.

The model training system 100 is configured to train a data matching model 112. According to embodiments of the present disclosure, the data matching model 112 is constructed to implement a data matching task. For an input data sample of one type, it is expected to find one or more data samples of another type that matches with the input data sample. Herein, different types of data samples are considered as different data modalities. The data matching implemented by the data matching model 112 is thus considered as a cross-modality data matching task. Specifically, the data matching model 112 is configured to determine whether an input data sample matches with respective data samples from a first dataset of a first type and a second dataset of a second type.

In some embodiments, the different types of data samples may include an image type and a text type. In an example, it may be expected to search for matching images given a text document or search for matching text documents given an image. In some embodiments, the different types of data samples may include different image types which may have visual contents in different styles. In an example, given an image of a certain type, it may be expected to search for matching images of a different type. An image and a text document are considered to match with each other if the text document contains text accurately describing the image. Two images are considered to match with each other if they present the same or similar visual contents. Two text documents may be considered to match with each other if they present the same or similar textual contents.

In the medical field, the different types of data samples may include medical types. In some embodiments, the different types may include a medical image type such as a Computed Tomography (CT)-based image, X-ray based image data, Positron Emission Tomography (PET)-based image, medical ultrasonography-based image, Nuclear Magnetic Resonance Imaging (NMRI)-based image, and/or any other images based on medical imaging technology. The different types of data samples may further include a medical text type as such a medical report describing observations/findings in a corresponding medical image, interpretation about a medical image, potential diagnostic information, medical recommendations, and/or the like. As a specific example, after taking a medical image of a patient, the radiologist may need to write a radiology report for the medical image, to describe his/her observations on the medical image. In this example, the medical image and the radiology report are generally considered to match with each other.

In some use cases, given a newly produced medical image, it may be desired to search for some matching medical images and/or matching medical reports. The matching medical images and/or textual reports can provide some guidance or recommendation to write an accurate high-quality report for the newly produced medical image. In some other use cases, some medical researchers may also want find as much as possible similar medical images and associated medical reports about a same disease or lesion. In either case, the data matching between medical image data and medical text data is useful.

In some embodiments, the different types may include different medical image types, including a CT-based image, X-ray based image, PET-based image, medical ultrasonography-based image, NMRI-based image, and/or any other images based on medical imaging technology. In some embodiments, the medical image type may include a dynamic image sequence which includes more than one static image. For example, some medical imaging examine may produce a series of medical images about a body part, and some key images may be selected for use in diagnose. In some cases, different medical imaging technologies can be applied for a same body part of a patient, to check whether there is any lesion on the body part. Different medical imaging technologies may have different complexity and costs, and lead to different accuracies in terms of diagnose. In some cases, the physicians may choose to first perform one type of medical imaging on a patient under examination, such as a cheaper or quicker one.

After obtaining a medical image of a first type for a patient under examination, the data matching process may utilize the medical image as an input to find matching images of a second type generated through another medical image technology (which is believed to have a higher accuracy). Among the found matching medical images, a pair of matching images of the first and second types can be associated with each other, for example, belong to a same patient. If it is observed from the matching images of the second type that some potential lesions occur, the physician may determine that the patient under examination may probably have similar lesions and then choose to apply the other medical image technology with more complexity but higher accuracy. Otherwise, if it is observed from the matching images of the second type that no potential lesion is found, the following complicated medical imaging procedure may be omitted for the patient. That is, the cross-modality data matching for medical images of different types can help determine whether further complicated examination is needed. As a result, the whole medical examination process can be accelerated, the medical resources can be reserved and the cost for the patients is also reduced.

Some examples of cross-modality data matching have been introduced above. It should be appreciated that many other types may be involved for the cross-modality data matching. It should be appreciated that and it can be applied in many other fields in addition to the medical field. The scope of the present disclosure is not limited in this regard.

To train the data matching model 112 to achieve the capability of performing cross-modality data matching, the model training system 110 obtains a first training dataset 101 comprising data samples of a first type and a second training dataset 102 of a second type. Each of the first and second datasets 101, 102 comprises a plurality of data samples of the respective type. In some embodiments, at least some of the data samples in the first training dataset 101 may be labeled or marked as matching with respective data samples in the second training dataset 102. The model training system 110 can perform the training process of the data matching model 112 according to a certain training objective, which will be described in detail below.

Initially, the data matching model 112 may be constructed to have a set of initial parameter values. During the training process, the set of parameter values of the data matching model 112 may be updated until the training objective is achieved. After the training process is completed, the trained data matching model 112 can be obtained, which may have a set of trained parameter values.

The trained data matching model 112 may be provided to the model applying system 120 to apply for new data samples. As illustrated in Fig. 1, the model applying system 120 obtains a target data sample 122 which may be of either the first type or the second type. The model applying system 120 have access to a first target dataset 131 including data samples of the first type and a second target dataset 132 including data samples of the second type. The data samples included in the first target dataset 131 of the first type may be different, the same, or partially the same as those included in the first training dataset 101 of the first type. Similarly, the data samples in the second target dataset 132 of the second type may be different, the same, or partially the same those included in the second training dataset 102, although their types are respectively the same. The model applying system 120 uses the trained data matching model 112 to generate a matching result 140, which indicates whether the target data sample 122 matches with any data samples in the first target dataset 131 and/or in the second target dataset 132.

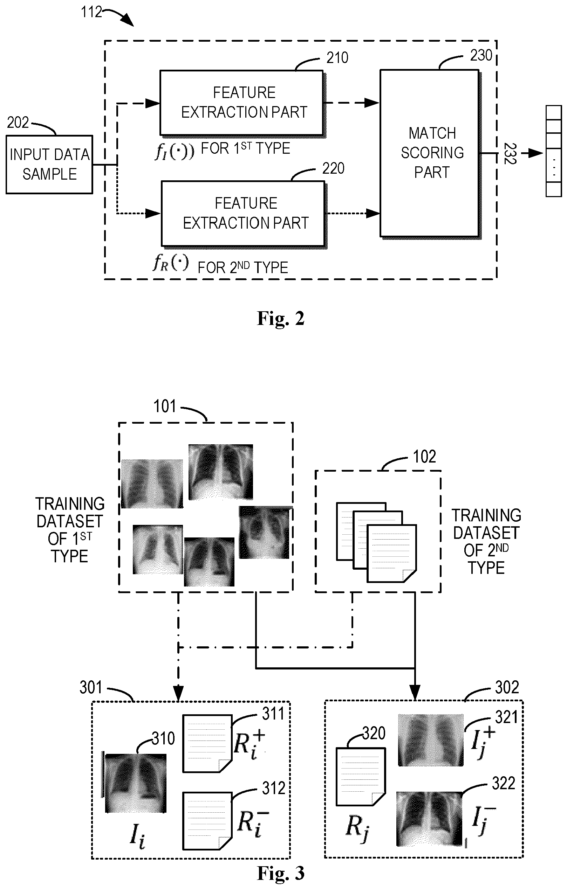

To better understand the operations in the data matching model 112 and its training and applying processes, example architecture of the data matching model 112 will be described first. Fig. 2 illustrates a block diagram of architecture of the data matching model 112 according to some embodiments of the present disclosure.

The data matching model 112 is a machine learning or deep learning model. The data matching model 112 may be constructed using various machine learning or deep learning model architectures. As illustrated in Fig. 2, the data matching model 112 comprises a feature extraction part 210 for the first type (represented as $f_I(\cdot)$) and a feature extraction part 220 for the second type (represented as $f_R(\cdot)$). The feature extraction part 210 is configured to extract a feature representation of the input data sample if an input data sample 202 to the data matching model 112 is of the first type, and the feature extraction part 220 is configured to extract a feature representation of the input data sample 202 if the input data sample 202 is of the second type. As used herein, a feature representation is in form of multi-dimension vector, to characterize the input data sample. In operation of the data matching model 112, the input data sample 202 may be input to either the feature extraction part 210 or the feature extraction part 220 depending on its type.

In some embodiments, the feature extraction part 210 may be constructed as a machine learning or deep learning model that is suitable to process or extract feature representations of data samples of the first type, and the feature extraction part 220 may be constructed as a machine learning or deep learning model that is suitable to process or extract feature representations of data samples of the second type. In an example, if the first type is an image type, the feature extraction part 210 may be constructed as a Convolutional Neural Network (CNN) model, or any other deep learning model that is suitable for processing image data. As another example, if the second type is a text type, the feature extraction part 210 may be constructed as a deep learning model suitable for nature language processing, such as a language model, a BERT model, an Auto-encoder model, a Long Short-Term Memory (LSTM) model, or any other deep learning model that is suitable for processing text data. In some embodiments, one or both of the feature extraction parts 210 and 220 may be pre-trained models which have initial parameter values determined through the pre-training processes.

As illustrated in Fig. 2, the data matching model 112 further includes a match scoring part 230. The match scoring part 230 is configured to receive the feature representation of the input data sample 202 from the feature extraction part 210 or 220, and determine respective matching scores between the input data sample 202 and respective data samples in a first dataset of the first type and/or a second dataset of the second type, based on the feature representation of the input data sample 202 and feature representations of the respective data samples in the first dataset and/or second dataset. The feature representations of the respective data samples in the first dataset may be extracted by the feature extraction part 210 and the feature representations of the respective data samples in the second dataset 220 may be extracted by the feature extraction part 220.

An output 232 of the match scoring part 230 may include the respective matching scores between the input data sample 202 and the respective data samples in one or both of the two datasets. Each matching score indicates a matching level between the input data sample 202 and a data sample in a dataset, to indicate whether the two data samples math with each other.

During the model training stage, data samples in the first training dataset 101 and the second training dataset 102 are input to the data matching model 112 and the matching scores between different data samples in the two training datasets are determined and used to update the set of parameter values of the data matching model 112, as will be discussed in detail later. During the model application stage, the target data sample 122 is input to the trained data matching model 112 and matching scores between the target data sample 122 and data samples in one or both of the first and second target datasets 131, 132 are determined.

Example embodiments of the model training stage for the data matching model 112 will be described first and then example embodiments of the model applying stage.

*Training of Data Matching Model*

According to embodiments of the present disclosure, in order to train the data matching model 112 to achieve the capability of cross-modality data matching, a triplet loss is utilized when determining the training objective for the data matching model 112. To calculate the triplet loss, data samples in the two training datasets 101 and 102 are organized as respective pairs of training data sample sets, each training data sample set being constructed as a triplet training sample comprising three data samples from the two training datasets 101 and 102.

Fig. 3 illustrates an example of constructing training sample sets for training the data matching model 112. In this example, it is assumed that the first training dataset 101 of the first type comprises medical images, and the second training dataset 102 of the second type comprises medical images. As illustrated, an *i*-th training sample set 301 comprises an anchor data sample 310 (represented as $I_i$) of the first type, a positive data sample 311 (represented as $R_i^+$) of the second type, and a negative data sample 312 (represented as $R_i^-$) of the second type. An *j*-th training sample set 302 comprises an anchor data sample 320 (represented as $R_j$) of the second type, a positive data sample 321 (represented as $I_j^+$) of the first type, and a negative data sample 322 (represented as $I_j^-$) of the first type.

In the training sample set 301, the anchor data sample $I_i$ is selected from the first training dataset 101 of the first type, and the positive and negative data samples $R_i^+$ and $R_i^-$ are selected from the second training dataset 102 of the second type. The "positive" data sample $R_i^+$ matches with the anchor data sample $I_i$, which means that the medical report of the positive data sample $R_i^+$ describes observations from the medical image of the anchor data sample $I_i$ in the example of Fig. 3. For example, the medical report and the medical images of a same patient obtained for a medical test may be considered to match with each other. The "negative" data sample $R_i^-$ mismatches with the anchor data sample $I_i$, which may mean that the medical report of the negative data sample $R_i^+$ does not contain observations from the medical image of the anchor data sample $I_i$.

Similarly, in the training sample set 302, the anchor data sample $R_j$ is selected from the second training dataset 102 of the second type, and the positive and negative data samples $I_j^+$ and $I_j^-$ are selected from the first training dataset 101 of the first type. The positive data sample $I_j^+$ matches with the anchor data sample $R_j$, while the negative data sample $I_j^-$ mismatches with the anchor data sample $R_j$.

For such training sample sets, for each data sample set, the model training may explore anchor data samples from one modality, together with positive and negative data samples from the other modality. To train the data matching model 112, a plurality of pairs of training data sample sets similar to those as illustrated in Fig. 3 may be constructed from the first and second training datasets 101, 102.

In some embodiments, the model training system 110 may obtain labelling information for the first and second training datasets 101 and 102, indicating which data sample in the first training dataset 101 matches and/or mismatches with which data sample(s) in the second training dataset 102. The model training system 110 may randomly select the anchor data sample $I_i$ from the first training dataset 101 and the anchor data sample $R_j$ from the second training dataset 102, and then select the corresponding positive and negative data samples based on the labelling information. In some embodiments, the labelling information may indicate only the matching of the data samples among the two training datasets 101, 102, and thus the negative data samples in each data sample set may be selected randomly from other data samples or may be computed via hard negative samples.

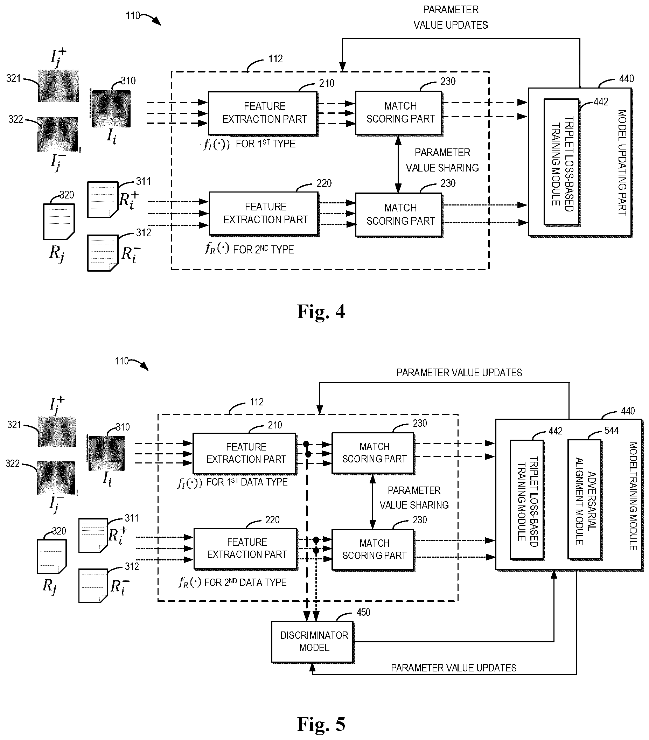

Reference is first made to Fig. 4, which illustrates a block diagram of the model training system 110 for training the data matching model 112 according to some embodiments of the present disclosure. The example architecture of the data matching model 112 in Fig. 2 is illustrated as an example here for the model training.

As briefly discussed above, during the training process, the set of parameter values of the data matching model 112 may be updated until the training objective is achieved. The model training system 110 comprises a model updating part 440 which is configured to train the data matching model 112, more specifically, to determine parameter value updates for the set of parameter values of the data matching model 112. Specifically, in the embodiments of Fig. 4, the model updating part 440 comprises a triplet loss-based training module 442 configured to train the data matching model 112 with at least a pair of training sample sets 301, 302 using a triplet loss, so as to meet the training objective.

To achieve the capability of cross-modality data matching, the training objective is configured so as to enable the trained data matching model 112 to determine that the positive data sample 311 $R_i^+$ matches with the anchor data sample 310 $I_i$ and the negative data sample 312 $R_i^-$ mismatches with the anchor data sample 310 $I_i$, as well as to determine that the positive data sample 321 $I_j^+$ matches with and the negative data sample 322 $I_j^-$ mismatches with the anchor data sample 320 $R_j$.

To meet the above training objective, the triplet loss-based training model 442 may be configured to construct a triplet loss function to optimize or update the data matching model 112. The triplet loss is to optimize the difference between an anchor data sample and a positive data sample as small as possible, and the difference between the anchor data samples and a negative data samples as large as possible. Generally, the match scoring part 230 is configured as a scoring function to determine a matching score between two data samples based on a distance between feature representations of the two data samples. The triplet loss is taken into account to mainly update the parameter values of the feature extraction parts 210, 220 ($f_I(\cdot)$) and $f_R(\cdot)$) so that they can generate feature representations with a small distance for matching data samples of two types as well as generate feature representations with a large distance for mismatching data samples of two types.

In some embodiments, the triplet loss function may be constructed as a loss function based on the following differences: a difference between the positive data sample 311 $R_i^+$ and the anchor data sample 310 $I_i$ and a difference between the negative data sample 312 $R_i^-$ and the anchor data sample 310 $I_i$ for the training sample set 301, and a difference between the positive data sample 321 $I_j^+$ and the anchor data sample 320 $R_j$ and a difference between the negative data sample 322 $I_j^-$ and the anchor data sample 320 $R_j$ for the training sample set 302. The difference between two data samples may be determined as a match scoring between the two data samples, to measure whether the two data samples match with each other or not.

During the training process, as illustrated in Fig. 4, the data samples in each of the two training sample sets 301, 302 may be input to the corresponding feature extraction parts 210, 220, depending on their types. The corresponding feature extraction parts 210, 220 extracts, with their current set of parameter values, feature representations of those data samples are provided to the match scoring part 230 to determine matching scores. More specifically, the match scoring part 230 determines a first matching score between the anchor data sample 310 $I_i$ and the positive data sample 311 $R_i^+$, a second matching score between the anchor data sample 310 $I_i$ and the negative data sample 312 $R_i^-$ for the training sample set 301, and a third matching score between the anchor data sample 320 $R_j$ and the positive data sample 321 $I_j^+$ and a fourth matching score between the anchor data sample 320 $R_j$ and the negative data sample 322 $I_j^-$ for the training sample set 302. In Fig. 4, although two instances of the match scoring parts 220 are illustrated, they may be considered as a same part with the shared parameter values. The triple loss function may be optimized by decreasing the sum of a first difference between the first matching score and the second matching score and a second difference between the third matching score and the fourth matching score, so as to meet the training objective.

In some embodiments, the triple loss function may be determined as follows:

$$\min \ell_{tri}(f_I, f_R) = \sum_i [d(f_I(I_i), f_R(R_I^+)) - d(f_I(I_i), f_R(R_I^-)) + \alpha]_+ \qquad (1)$$
$$+ \sum_j [d(f_R(R_j), f_I(I_j^+)) - d(f_R(R_j), f_I(I_j^-)) + \alpha]_+$$

In Equation (1), $i$ represents the $i$th training sample set with an anchor data sample of the first type, and $j$ represents the $j$th training sample set with an anchor data sample of the second type. $f_I(I_i)$ represents the feature representation of the anchor data sample 310 $I_i$ extracted by the feature extraction part 210; $f_R(R_i^+)$ represents the feature representation of the positive data sample 311 $R_i^+$ extracted by the feature extraction part 220; $f_R(R_i^-)$ represents the feature representation of the negative data sample 312 $R_i^-$ extracted by the feature extraction part 220; $f_R(R_j)$ represents the feature representation of the anchor data sample 320 $R_j$ extracted by the feature extraction part 220, $f_I(I_j^+)$ represents the feature representation of the positive data sample 321 $I_j^+$ extracted by the feature extraction part 210, $f_I(I_j^-)$ represents the feature representation of the negative data sample 322 $I_j^-$ extracted by the feature extraction part 210.

In Equation (1), $d(\cdot)$ represents a difference or a distance between the feature representations of the two data samples, which may indicate the matching score of the two data samples. In Equation (1), the item $\alpha$ represents a minimal gap between the difference for the anchor data sample and the positive data sample and the difference for the anchor data sample and the negative data sample. The value of $\alpha$ is a hyper-parameter which may be predetermined or configured. In Equation (1), $\lfloor \ \rfloor_+$ represents that if a resulting value within $\lfloor \ \rfloor$ is larger than zero, this value may be determined as a triplet loss; otherwise, if the resulting value within $\lfloor \ \rfloor$ is smaller than or equal to zero, than the triplet loss is zero.

If the training objective for the data matching is based on the triplet loss in Equation (1), the training objective is to optimize or minimize the triplet loss. The triplet loss-based training module 442 may determine parameter value updates for the respective parts of the data matching model 112 such that the triplet loss determined based on the set of updated parameter values is decreased and finally minimized and the training objective is met. The triplet loss-based training module 442 may utilize various model training methods, such as random gradient descent and its variants to determine the parameter value updates. As mentioned above, the set of parameter values for the data matching model 112 may be updated iteratively using training sample sets constructed from the first and second training datasets 101, 102, so as to meet the training objective.

In embodiments of the present disclosure, by simultaneously considering two training sample sets with anchor data samples from the two modalities, the data matching model 112 can be well trained to determine matching data samples for an input data sample of any of the two modalities (or types).

In some embodiments, in order to well correlate different encoded distributions from different modalities, an adversarial strategy may be further applied during the training process of the data matching model 112. The adversarial strategy is leveraged to conduct cross-modality alignment, resulting in the in-distinguishability between data samples of different modalities, such as medical images and medical reports. In order to match different feature representations from different modalities, it is expected that the feature representations of the data samples in different modalities may be learnt to well aligned and correlated between different modalities, reducing the modality invariance for the further improvement of cross-modality matching. The cross-modality alignment may be implemented by a discriminator model in a generative adversarial network (GAN), which makes use of the discriminator model to confuse heterogeneous modalities, for example, the medical images and reports.

Fig. 5 illustrates a block diagram of the model training system 110 for training the data matching model 112 according to the embodiments related to the adversarial strategy. As illustrated in Fig. 5, to apply the adversarial strategy, the model training system 110 comprises a discriminator model 450 (represented as "$D$"). The discriminator model 450 serves as an adversary to conduct adversarial domain adaptation, which aims to fail to distinguish whether a feature representation is extracted from a data sample of the first type or a data sample of the second type. More specifically, the discriminator model 450 may be constructed to attempt to discriminate modalities of data samples, for example, to determine whether a data sample is the first type (e.g., a medical image) or the second type (e.g., a medical report). In particular, the discriminator model 450 is constructed to determine a probability of a data sample being of the first type or of the second type based on a feature representation of the data sample. The feature representation of the data sample may be extracted by the feature extraction part 210 or 220, depending on its type.

The discriminator model 450 may be a discriminator from a GAN. The discriminator model 450 is also configured with a set of parameter values, to process the input feature representation of a data sample and output the probability. The data matching model may be jointly trained with the discriminator model 450. The training objective for the data matching model 112 may be further configured to cause the discriminator model 450 to fail to discriminate types of the matching data samples in the first and second training datasets 101, 102. For example, for the training sample sets 301 and 302, the training objective is configured to cause the discriminator model 450 to fail to discriminate the types of the anchor data sample 310 $I_i$ and the positive data sample 311 $R_i^+$ and/or the types of the anchor data sample 320 $R_j$ and the positive data sample 321 $I_j^+$.

To achieve this, the feature extraction parts 210 and 220 may be trained to generate aligned feature representations for the matching data samples of the different types so that the discriminator model 450 may not be able to distinguish the types of the data samples. In other words, with the instruction of the discriminator model 450, the feature extraction parts 210 and 220 may be enhanced to extract more aligned feature representations for a pair of matching data samples of different types, such that the discriminator model 450 cannot distinguish whether a data sample is from the first training dataset 101 of the first type or the second training dataset 102 of the second type.

The model updating part 440 comprises an adversarial alignment module 442 configured to jointly train the data matching model 112 and the discriminator model 450 based on the adversarial strategy, together with the triplet loss-based training module 442. In some embodiments, the adversarial alignment module 442 may employ the min-max optimization to converge to the training objective of causing the discriminator model 450 to fail to discriminate types of matching data samples. The min-max optimization may be formulated as follows:

$$\min_{(f_I, f_R)} \max_{\mathcal{D}} \ell_{adv} = E_{(i,r) \sim p(I,R)} \big[ \log \mathcal{D}(f_I(i)) + \log(1 - \mathcal{D}(f_R(r))) \big] \qquad (2)$$

In Equation (2), $p(I, R)$ represents a set of matching data samples from the two types in the training datasets 101, 102; $i$ represents the $i$th data sample of the first type, and $r$ represents the $r$th data sample of the second type that matches with the ith data sample; $\mathcal{D}$ ($f_I(i)$) represents an output probability of the ith data sample being the first type by the discriminator model 450; (1-$\mathcal{D}$ ($f_R(r)$) represents an output probability of the $r$th data sample being the first type by the discriminator model 450. During the training, the output probability by the discriminator model 450 is determined based on the feature representation of the corresponding data sample extracted by the feature extraction part 210 $f_I(\cdot)$ or the feature extraction part 220 $f_R(\cdot)$ with their current parameter values.

Based on the min-max optimization in Equation (2), the adversarial alignment module 442 may determine parameter value updates for the discriminator model 450 so as to maximize $D(f_I(i))$ and $D(f_R(r))$, and parameter value updates for the feature extraction parts 210, 220, so as to minimize $\max_{\mathcal{D}} \ell_{adv}$ with respect to ($f_I$, $f_R$). During the training, the discriminator model 450 and the feature extraction parts 210, 220 may be both dynamically updated/trained until they reach the min-max optimization. That is, a feature representation of a data sample generated by the feature extraction part 210 or 220 are indistinguishable (or a close to indistinguishable as possible) from a feature representation of a data sample generated by the feature extraction part 220 or 210 through the discriminator model 450. The training objective may be met if both the triplet loss function is optimized and the min-max optimization is achieved.

*Applying of Data Matching Model*

The embodiments related to the training of the data matching model 112 have been described above. After the training process is completed, the trained data matching model 112 may be determined with a set of trained parameter values. The trained data matching model 112 may be utilized to predict matching data samples in different modalities. In the embodiments related to the adversary strategy, the trained discriminator model 450 may be discarded as it is generally useless in the following model applying stage.

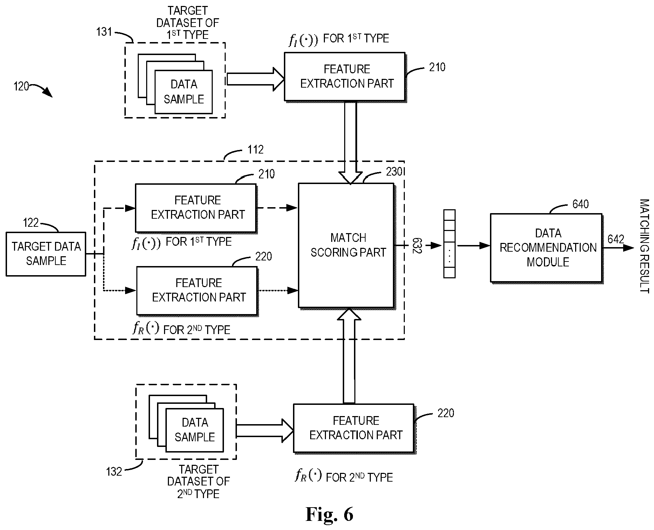

Fig. 6 illustrates a block diagram of the model applying system 120 for performing data matching according to some embodiments of the present disclosure. The trained data matching model 112 determined by the model training system 110 may be utilized in the model applying system 120.

As illustrated, it is assumed that the trained data matching model 112 is used to determine one or more matching data samples for the input target data sample 122. Depending on the type of the target data sample 122, it may be input to either the feature extraction part 210 for the first type or the feature extraction part 220 for the second type, to generate a target feature representation of the target data sample 122. The target feature representation of the target data sample 122 may be provided to the match scoring part 230 in the trained data matching model 112.

The match scoring part 230 may determine target matching scores between the target data sample and respective data samples included in the first target dataset 131 of the first type and/or respective data samples included in the second target dataset 132 of the second type. The matching scoring part 230 may also obtain respective feature representations of the data samples included in the first target dataset 131 and/or the second target dataset 132, and determine the target matching scores based on the feature representation of the target data sample 122 and the respective feature representations of the data samples. As illustrated in Fig. 6, the feature representations of the data samples included in the first target dataset 131 may be determined using the feature extraction part 210 for the first type, and the feature representations of the data samples included in the second target dataset 132 may be determined using the feature extraction part 220 for the second type. In some embodiments, the feature representations of the data samples in the first and second target datasets 131, 132 may be determined in advance and stored for use in determining the matching scores for the input target data sample 122.

The matching scoring part 230 may provide an output 632 to indicate the determined target matching scores. The output 632 may be provided to a data recommendation module 640 included in the model applying system 120. The data recommendation module 640 is configured to provide a matching result 642, which comprises at least one indication of at least one of the data samples in the first training dataset 131 and/or the second training dataset 132 based on the determined target matching scores. For example, the data recommendation module 640 may select a predetermined number of data samples (which may be larger than or equal to one) having the highest target matching scores with the target data sample 122. Alternatively, the data recommendation module 640 may select one or more data samples having their target matching scores exceeding a predetermined threshold score. The data sample(s) indicated by the data recommendation module 640 may be provided or presented as a recommendation to the user. For example, by inputting a target medical image to the model applying system 120, the physician may be recommended with one or more medical reports stored in the database which are determined to match with the target medical image, to facilitate the report writing for the target medical image.

Since the data matching model 112 is trained with the triplet loss determined from two kinds of training sample sets 301, 302, it is capable of determining matching data samples of a different type from the input target data sample 122 or matching data samples of the sample type as the input target data sample 122. For example, the target data sample 122 is of the first type, the data matching model 112 is capable of determining matching scores between the target data sample 122 and data samples in the second target dataset 132 of the second type different from the first type. On the other hand, the data matching model 112 is capable of determining matching scores between the target data sample 122 and data samples in the first target dataset 131 of the same first type.

In some embodiments, the user may indicate matching data samples of the same or different types are expected for the target data sample 122. The model applying system 120 may receive a user input indicating at least one of the first and second types. According to the user input, the model applying system 120 may determine data samples in which one or both of the first and second target datasets 131, 132 are to be considered. The model applying system 120 may cause the data matching model 112 to determine matching scores between the target data sample and data samples included in the determined target dataset(s). In this way, the user may be able to control the type(s) of expected matching data samples.

*Example Processes*

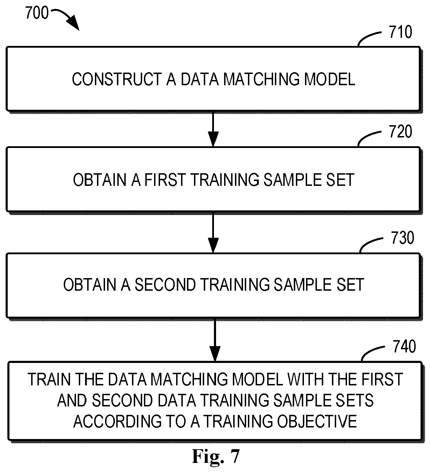

Fig. 7 illustrates a flowchart of an example method 700 according to some embodiments of the present disclosure. The method 700 can be implemented by the model training system in Fig. 1.

At block 710, the model training system 110 constructs a data matching model to be configured to determine whether an input data sample matches with a reference data sample included in either one of a first dataset of a first type and a second dataset of a second type.

In some embodiments, the first and the second types comprise medical data types.

In some embodiments, the first type comprises a medical text type, and the second type comprises a medical image type. In some embodiments, the first type comprises a first medical image type, and the second type comprises a second medical image type.

At block 720, the model training system 110 obtains a first training sample set comprising a first anchor data sample of a first type, and a first positive data sample and a first negative data sample of a second type.

At block 730, the model training system 110 obtains a second training sample set comprising a second anchor data sample of the second type, and a second positive data sample and a second negative data sample of the first type.

At block 740, the model training system 110 trains the data matching model with the first and second training sample sets according to a training objective. The training objective is configured to enable the trained data matching model to determine that the first positive data sample matches with and the first negative data sample mismatches with the first anchor data sample, and to determine that the second positive data sample matches with and the second negative data sample mismatches with the second anchor data sample.

In some embodiments, to train the data matching model, the model training system 110 determines, using the data matching model, a first matching score between the first anchor data sample and the first positive data sample, a second matching score between the first anchor data sample and the first negative data sample, a third matching score between the second anchor data sample and the second positive data sample, and a fourth matching score between the second anchor data sample and the second negative data sample. The model training system 110 updates the data matching model by decreasing a sum of a first difference between the first matching score and the second matching score and a second difference between the third matching score and the fourth matching score to meet the training objective.

In some embodiments, the data matching model comprises: a first feature extraction part configured to extract a feature representation of the input data sample if the input data sample is of the first type, a second feature extraction part configured to extract the feature representation of the input data sample if the input data sample is of the second type, and a match scoring part configured to determine a matching score between the input data sample and the reference data sample based on the feature representation of the input data and a feature representations of the reference data sample.

In some embodiments, to train the data matching model, the model training system 110 constructs a discriminator model to be configured to determine a probability of a data sample being of the first type or of the second type based on a feature representation of the data sample. The model training system 110 jointly trains the data matching model and the discriminator model with the first and second training sample sets according to the training objective. In these embodiments, the training objective is further configured to cause the discriminator model to fail to discriminate the types of the first anchor data sample and the first positive data sample and/or the types of the second anchor data sample and the second positive data sample.

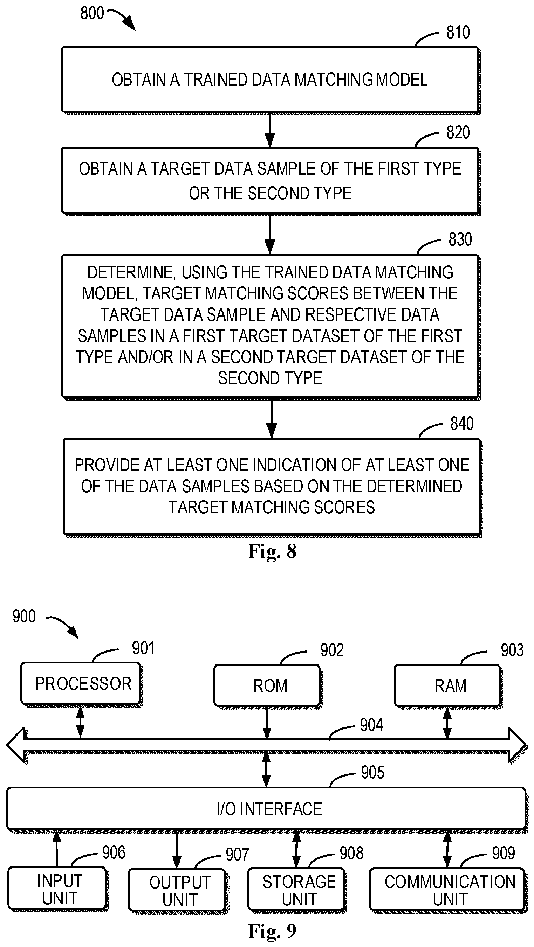

In some embodiments, the data matching model trained by the process 700 may be provide for use, as will be described below with reference to Fig. 8.

Fig. 8 illustrates a flowchart of an example method according to some other embodiments of the present disclosure. The method 800 can be implemented by the model applying system 120 in Fig. 2.

At block 810, the model applying system 120 obtains the trained data matching model.

At block 820, the model applying system 120 obtains a target data sample of the first type or the second type. At block 830, the model applying system 120 determines, using the trained data matching model, target matching scores between the target data sample and respective data samples included in a first target dataset of the first type and/or in a second target dataset of the second type. At block 840, the model applying system 120 provides at least one indication of at least one of the data samples based on the determined target matching scores.

In some embodiments, to determine the target matching scores, the model applying system 120 receives a user input indicating at least one of the first and second types, and determines at least one of the first and second target datasets of the at least one of the first and second types indicated by the user input. The model applying system 120 determines the target matching scores between the target data sample and data samples included in the determined at least one of the first and second target datasets.

*Example System/Device*

Fig. 9 illustrates a block diagram of an example computing system/device 900 suitable for implementing example embodiments of the present disclosure. The system/device 900 can be implemented as or implemented in the model training system 110 and/or the model applying system 120 in Fig. 1. The system/device 900 may be a general-purpose computer, a physical computing device, or a portable electronic device, or may be practiced in distributed cloud computing environments where tasks are performed by remote processing devices that are linked through a communication network. The system/device 900 can be used to implement the method 700 of Fig. 7 and/or the method 800 of Fig. 8.

As depicted, the system/device 900 includes a processor 901 which is capable of performing various processes according to a program stored in a read only memory (ROM) 902 or a program loaded from a storage unit 908 to a random access memory (RAM) 903. In the RAM 903, data required when the processor 901 performs the various processes or the like is also stored as required. The processor 901, the ROM 902 and the RAM 903 are connected to one another via a bus 904. An input/output (I/O) interface 905 is also connected to the bus 904.

The processor 901 may be of any type suitable to the local technical network and may include one or more of the following: general purpose computers, special purpose computers, microprocessors, digital signal processors (DSPs), graphic processing unit (GPU), co-processors, and processors based on multicore processor architecture, as non-limiting examples. The system/device 900 may have multiple processors, such as an application-specific integrated circuit chip that is slaved in time to a clock which synchronizes the main processor.

A plurality of components in the system/device 900 are connected to the I/O interface 905, including an input unit 906, such as keyboard, a mouse, or the like; an output unit 907 including a display such as a cathode ray tube (CRT), a liquid crystal display (LCD), or the like, and a loudspeaker or the like; the storage unit 908, such as disk and optical disk, and the like; and a communication unit 909, such as a network card, a modem, a wireless transceiver, or the like. The communication unit 909 allows the system/device 900 to exchange information/data with other devices via a communication network, such as the Internet, various telecommunication networks, and/or the like.

The methods and processes described above, such as the method 700 and/or method 800, can also be performed by the processor 901. In some embodiments, the method 700 and/or method 800 can be implemented as a computer software program or a computer program product tangibly included in the computer readable medium, e.g., storage unit 908. In some embodiments, the computer program can be partially or fully loaded and/or embodied to the system/device 900 via ROM 902 and/or communication unit 909. The computer program includes computer executable instructions that are executed by the associated processor 901. When the computer program is loaded to RAM 903 and executed by the processor 901, one or more acts of the method 700 and/or method 800 described above can be implemented. Alternatively, processor 901 can be configured via any other suitable manners (e.g., by means of firmware) to execute the method 700 and/or method 800 in other embodiments.

In some example embodiments of the present disclosure, there is provided a computer program product comprising instructions which, when executed by a processor of an apparatus, cause the apparatus to perform steps of any one of the methods described above.

In some example embodiments of the present disclosure, there is provided a computer readable medium comprising program instructions for causing an apparatus to perform at least steps of any one of the methods described above. The computer readable medium may be a non-transitory computer readable medium in some embodiments.

In an eighth aspect, example embodiments of the present disclosure provide a computer readable medium comprising program instructions for causing an apparatus to perform at least the method in the second aspect described above. The computer readable medium may be a non-transitory computer readable medium in some embodiments.

Generally, various example embodiments of the present disclosure may be implemented in hardware or special purpose circuits, software, logic or any combination thereof. Some aspects may be implemented in hardware, while other aspects may be implemented in firmware or software which may be executed by a controller, microprocessor or other computing device. While various aspects of the example embodiments of the present disclosure are illustrated and described as block diagrams, flowcharts, or using some other pictorial representations, it will be appreciated that the blocks, apparatuses, systems, techniques, or methods described herein may be implemented in, as non-limiting examples, hardware, software, firmware, special purpose circuits or logic, general purpose hardware or controller or other computing devices, or some combination thereof.

The present disclosure also provides at least one computer program product tangibly stored on a non-transitory computer readable storage medium. The computer program product includes computer-executable instructions, such as those included in program modules, being executed in a device on a target real or virtual processor, to carry out the methods/processes as described above. Generally, program modules include routines, programs, libraries, objects, classes, components, data structures, or the like that perform particular tasks or implement particular abstract types. The functionality of the program modules may be combined or split between program modules as desired in various embodiments. Computer-executable instructions for program modules may be executed within a local or distributed device. In a distributed device, program modules may be located in both local and remote storage media.

The computer readable medium may be a computer readable signal medium or a computer readable storage medium. A computer readable medium may include but is not limited to an electronic, magnetic, optical, electromagnetic, infrared, or semiconductor system, apparatus, or device, or any suitable combination of the foregoing. More specific examples of the computer readable storage medium would include an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing.

Computer program code for carrying out methods disclosed herein may be written in any combination of one or more programming languages. The program code may be provided to a processor or controller of a general purpose computer, special purpose computer, or other programmable data processing apparatus, such that the program codes, when executed by the processor or controller, cause the functions/operations specified in the flowcharts and/or block diagrams to be implemented. The program code may execute entirely on a computer, partly on the computer, as a stand-alone software package, partly on the computer and partly on a remote computer or entirely on the remote computer or server. The program code may be distributed on specially-programmed devices which may be generally referred to herein as "modules". Software component portions of the modules may be written in any computer language and may be a portion of a monolithic code base, or may be developed in more discrete code portions, such as is typical in object-oriented computer languages. In addition, the modules may be distributed across a plurality of computer platforms, servers, terminals, mobile devices and the like. A given module may even be implemented such that the described functions are performed by separate processors and/or computing hardware platforms.

While operations are depicted in a particular order, this should not be understood as requiring that such operations be performed in the particular order shown or in sequential order, or that all illustrated operations be performed, to achieve desirable results. In certain circumstances, multitasking and parallel processing may be advantageous. Likewise, while several specific implementation details are contained in the above discussions, these should not be construed as limitations on the scope of the present disclosure, but rather as descriptions of features that may be specific to particular embodiments. Certain features that are described in the context of separate embodiments may also be implemented in combination in a single embodiment. Conversely, various features that are described in the context of a single embodiment may also be implemented in multiple embodiments separately or in any suitable subcombination.

Although the present disclosure has been described in languages specific to structural features and/or methodological acts, it is to be understood that the present disclosure defined in the appended claims is not necessarily limited to the specific features or acts described above. Rather, the specific features and acts described above are disclosed as example forms of implementing the claims.

**Claims**

1. A computer-implemented method, comprising:

   constructing a data matching model to be configured to determine whether an input data sample matches with a reference data sample included in either one of a first dataset of a first type and a second dataset of a second type;
   obtaining a first training sample set comprising a first anchor data sample of a first type, and a first positive data sample and a first negative data sample of a second type;
   obtaining a second training sample set comprising a second anchor data sample of the second type, and a second positive data sample and a second negative data sample of the first type; and
   training the data matching model with the first and second training sample sets according to a training objective, the training objective configured to enable the trained data matching model to determine that the first positive data sample matches with and the first negative data sample mismatches with the first anchor data sample, and to determine that the second positive data sample matches with and the second negative data sample mismatches with the second anchor data sample.

2. The method of claim 1, wherein the first and the second types comprise medical data types.

3. The method of claim 2, wherein the first type comprises a medical text type, and the second type comprises a medical image type, or
wherein the first type comprises a first medical image type, and the second type comprises a second medical image type.

4. The method of any of claims 1 to 3, wherein training the data matching model comprises:

determining, using the data matching model, a first matching score between the first anchor data sample and the first positive data sample, a second matching score between the first anchor data sample and the first negative data sample, a third matching score between the second anchor data sample and the second positive data sample, and a fourth matching score between the second anchor data sample and the second negative data sample; and
updating the data matching model by decreasing a sum of a first difference between the first matching score and the second matching score and a second difference between the third matching score and the fourth matching score to meet the training objective.

5. The method of any of claims 1 to 4, wherein the data matching model comprises:

a first feature extraction part configured to extract a feature representation of the input data sample if the input data sample is of the first type,
a second feature extraction part configured to extract the feature representation of the input data sample if the input data sample is of the second type, and
a match scoring part configured to determine a matching score between the input data sample and the reference data sample based on the feature representation of the input data and a feature representations of the reference data sample.

6. The method of any of claims 1 to 5, wherein training the data matching model comprises:

constructing a discriminator model to be configured to determine a probability of a data sample being of the first type or of the second type based on a feature representation of the data sample; and
jointly training the data matching model and the discriminator model with the first and second training sample sets according to the training objective, the training objective further configured to cause the discriminator model to fail to discriminate the types of the first anchor data sample and the first positive data sample and/or the types of the second anchor data sample and the second positive data sample.

7. The method of any of claims 1 to 6, further comprising:

obtaining a target data sample of the first type or the second type;
determining, using the trained data matching model, target matching scores between the target data sample and respective data samples included in a first target dataset of the first type and/or in a second target dataset of the second type; and
providing at least one indication of at least one of the data samples based on the determined target matching scores.

8. The method of claim 7, wherein determining the target matching scores comprises:

receiving a user input indicating at least one of the first and second types;
determining at least one of the first and second target datasets of the at least one of the first and second types indicated by the user input; and
determining the target matching scores between the target data sample and data samples included in the determined at least one of the first and second target datasets.

9.  A computing system comprising:

    at least one processor; and
    at least one memory comprising computer readable instructions which, when executed by the at least one processor of the electronic device, cause the electronic device to perform acts comprising:

        constructing a data matching model to be configured to determine whether an input data sample matches with a reference data sample included in either one of a first dataset of a first type and a second dataset of a second type;
        obtaining a first training sample set comprising a first anchor data sample of a first type, and a first positive data sample and a first negative data sample of a second type;
        obtaining a second training sample set comprising a second anchor data sample of the second type, and a second positive data sample and a second negative data sample of the first type; and
        training the data matching model with the first and second training sample sets according to a training objective, the training objective configured to enable the trained data matching model to determine that the first positive data sample matches with and the first negative data sample mismatches with the first anchor data sample, and to determine that the second positive data sample matches with and the second negative data sample mismatches with the second anchor data sample.

10. The computing system of claim 9, wherein training the data matching model comprises:

    determining, using the data matching model, a first matching score between the first anchor data sample and the first positive data sample, a second matching score between the first anchor data sample and the first negative data sample, a third matching score between the second anchor data sample and the second positive data sample, and a fourth matching score between the second anchor data sample and the second negative data sample; and
    updating the data matching model by decreasing a sum of a first difference between the first matching score and the second matching score and a second difference between the third matching score and the fourth matching score to meet the training objective.

11. The computing system of any of claims 9 and 10, wherein the data matching model comprises:

    a first feature extraction part configured to extract a feature representation of the input data sample if the input data sample is of the first type,
    a second feature extraction part configured to extract the feature representation of the input data sample if the input data sample is of the second type, and
    a match scoring part configured to determine a matching score between the input data sample and the reference data sample based on the feature representation of the input data and a feature representations of the reference data sample.

12. The computing system of any of claims 9 to 11, wherein training the data matching model comprises:

    constructing a discriminator model to be configured to determine a probability of a data sample being of the first type or of the second type based on a feature representation of the data sample; and
    jointly training the data matching model and the discriminator model with the first and second training sample sets according to the training objective, the training objective further configured to cause the discriminator model to fail to discriminate the types of the first anchor data sample and the first positive data sample and/or the types of the second anchor data sample and the second positive data sample.

13. The computing system of any of claims 9 to 12, wherein the acts further comprise:

    obtaining a target data sample of the first type or the second type;
    determining, using the trained data matching model, target matching scores between the target data sample and respective data samples included in a first target dataset of the first type and/or in a second target dataset of the second type; and
    providing at least one indication of at least one of the data samples based on the determined target matching scores.

**14.** The computing system of any of claims 9 to 13, wherein the first type comprises a medical text type, and the second type comprises a medical image type, or
wherein the first type comprises a first medical image type, and the second type comprises a second medical image type.

**15.** A computer readable medium comprising instructions which, when executed by at least one processor, cause the at least one processor to perform the method according to claims 1 to 8.

**Fig. 1**

**Fig. 2**

**Fig. 3**

PARAMETER
VALUE UPDATES

110

112

321 $I_j^+$

322

310 $I_i$

$I_j^-$

320 311 $R_i^+$

$R_j$ 312 $R_i^-$

210 FEATURE EXTRACTION PART
$f_I(\cdot))$ FOR 1ST TYPE

230 MATCH SCORING PART

PARAMETER VALUE SHARING

220 FEATURE EXTRACTION PART
$f_R(\cdot)$ FOR 2ND TYPE

230 MATCH SCORING PART

440

442 TRIPLET LOSS-BASED TRAINING MODULE

MODEL UPDATING PART

**Fig. 4**

PARAMETER VALUE UPDATES

110

112

321 $I_j^+$

322

310 $I_i$

$I_j^-$

320 311 $R_i^+$

$R_j$ 312 $R_i^-$

210 FEATURE EXTRACTION PART
$f_I(\cdot))$ FOR 1ST DATA TYPE

230 MATCH SCORING PART

PARAMETER VALUE SHARING

220 FEATURE EXTRACTION PART
$f_R(\cdot)$ FOR 2ND DATA TYPE

230 MATCH SCORING PART

440

442 TRIPLET LOSS-BASED TRAINING MODULE

544 ADVERSARIAL ALIGNMENT MODULE

MODEL TRAINING MODULE

450 DISCRIMINATOR MODEL

PARAMETER VALUE UPDATES

**Fig. 5**

19

Fig. 6

700

710

CONSTRUCT A DATA MATCHING MODEL

720

OBTAIN A FIRST TRAINING SAMPLE SET

730

OBTAIN A SECOND TRAINING SAMPLE SET

740

TRAIN THE DATA MATCHING MODEL WITH THE FIRST AND SECOND DATA TRAINING SAMPLE SETS ACCORDING TO A TRAINING OBJECTIVE

Fig. 7

800

810

OBTAIN A TRAINED DATA MATCHING MODEL

820

OBTAIN A TARGET DATA SAMPLE OF THE FIRST TYPE
OR THE SECOND TYPE

830

DETERMINE, USING THE TRAINED DATA MATCHING
MODEL, TARGET MATCHING SCORES BETWEEN THE
TARGET DATA SAMPLE AND RESPECTIVE DATA
SAMPLES IN A FIRST TARGET DATASET OF THE FIRST
TYPE AND/OR IN A SECOND TARGET DATASET OF THE
SECOND TYPE

840

PROVIDE AT LEAST ONE INDICATION OF AT LEAST ONE
OF THE DATA SAMPLES BASED ON THE DETERMINED
TARGET MATCHING SCORES

**Fig. 8**

900

901
PROCESSOR

902
ROM

903
RAM

904

905

I/O INTERFACE

906
INPUT
UNIT

907
OUTPUT
UNIT

908
STORAGE
UNIT

909
COMMUNICATION
UNIT

**Fig. 9**

**EUROPEAN SEARCH REPORT**

Application Number

EP 22 17 2557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/365727 A1 (AGGARWAL PRANAV VINEET [US] ET AL) 25 November 2021 (2021-11-25) * paragraphs [0024], [0028], [0029], [0057], [0051] * ----- | 1-15 | INV. G16H30/20 G16H30/40 |
| A | CN 113 836 333 A (BEIJING BAIDU NETCOM SCI & TECH CO LTD) 24 December 2021 (2021-12-24) * paragraphs [0005] - [0008], [034f], [0105] * ----- | 1-15 | |
| A | US 2020/097604 A1 (LEE KUANG-HUEI [US] ET AL) 26 March 2020 (2020-03-26) * paragraphs [0039], [0089] * ----- | 1-15 | |
| A | CN 111 382 748 A (BEIJING XIAOMI SONGGUO ELECT CO LTD) 7 July 2020 (2020-07-07) * the whole document * ----- | 1-15 | |
| A | CN 113 343 705 A (UNIV SHANDONG) 3 September 2021 (2021-09-03) * the whole document * ----- | 1-15 | TECHNICAL FIELDS SEARCHED (IPC) G16H |

The present search report has been drawn up for all claims

| Place of search | Date of completion of the search | Examiner |
|---|---|---|
| Munich | 21 October 2022 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 2557

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2022

| Patent document cited in search report | | Publication date | Patent family member(s) | | Publication date |
|---|---|---|---|---|---|
| US 2021365727 | A1 | 25-11-2021 | AU 2020202021 | A1 | 17-12-2020 |
| | | | CN 112015940 | A | 01-12-2020 |
| | | | DE 102020001790 | A1 | 03-12-2020 |
| | | | US 2020380298 | A1 | 03-12-2020 |
| | | | US 2021365727 | A1 | 25-11-2021 |
| CN 113836333 | A | 24-12-2021 | NONE | | |
| US 2020097604 | A1 | 26-03-2020 | NONE | | |
| CN 111382748 | A | 07-07-2020 | NONE | | |
| CN 113343705 | A | 03-09-2021 | NONE | | |

For more details about this annex : see Official Journal of the European Patent Office, No. 12/82

EPO FORM P0459